# EUROPEAN PATENT SPECIFICATION

(11) **EP 3 151 811 B1**
(45) Date of publication and mention of the grant of the patent: **18.09.2019**
(21) Application number: 15806007.9
(22) Date of filing: 04.06.2015
(51) Int. Cl.: A61K 8/89, A61K 8/19, A61K 8/92, A61K 8/30, A61Q 5/00, A61Q 19/00, A61K 8/26, A61Q 1/02, A61Q 1/10, A61Q 5/06, A61K 8/891

(54) **COMPOSITIONS AND METHODS FOR PROVIDING LONG WEARING FILM TO KERATIN SURFACES**
ZUSAMMENSETZUNGEN UND VERFAHREN ZUR BEREITSTELLUNG EINES HOCHVERSCHLEISSFESTEN FILMS AUF KERATINOBERFLÄCHEN
COMPOSITIONS ET PROCÉDÉS POUR FORMER UN FILM À LONGUE TENUE SUR DES SURFACES KÉRATINIQUES

(30) Priority: 09.06.2014 US 201414299518
(43) Date of publication of application: 12.04.2017
(73) Proprietor: ELC Management LLC, Melville, NY 11747 (US)
(72) Inventor: STEPNIEWSKI, George, Melville, New York 11747 (US); SERRANO-GODOY, Milagro, Douglaston, New York 11362 (US); MAROTTA, Paul, Farmingdale, New York 11735 (US)
(74) Representative: Dehns
(86) International application number: PCT/US2015/034223
(87) International publication number: WO 2015/191365

(56) References cited:
- WO-A1-03/086333
- WO-A1-2014/149869
- JP-B2- 2 805 228
- US-A1- 2007 014 744
- US-A1- 2012 258 055
- DATABASE GNPD [Online] MINTEL; June 2013 (2013-06), "No Smudge Mascara", XP002774254, Database accession no. 2084583

## Description

### Technical Field

This application is in the field of cosmetic compositions for providing long wearing color tint to hair such as eyelashes, eyebrows, or hair.

### Background of the Invention

There are many instances where consumers want to apply color to keratin surfaces that lasts longer than a few hours or a day. For example, individuals that have very light hair often have light eyelashes and eyebrows too, and where darkening the lashes and brows would improve appearance. Other examples include individuals with graying hair who may want to touch up spots showing gray between dye sessions, and where the touch up color lasts more than a day. Similarly, some individuals want to darken eyebrows or hide gray. Accordingly, keratin fiber coloration that lasts longer than a day is very desirable in many instances.

Mintel (record ID 2084583) discloses an aqueous long-lasting mascara based on several waxes, volatile solvents, crosslinked organosiloxane film former, hectorite and quaternium-90 bentonite.

Lash tints that apply color to lashes that lasts more than a day are known. However, one problem with these lash tints is that the resin used to provide the desired long wearing property is quite brittle. If these resins are not plasticized with non-volatile oils the resulting lash tint tends to flake, or break off the lashes in pieces. In addition, the brittle resin causes the coated lashes to be more rigid which can promote lash breakage. On the other hand, if the formula contains ingredients that plasticize the resin, this compromises wear and adhesion. Gloss is also an important and desirable feature of long wearing color tints. Thus, it is important to maximize the benefits of the composition in each of the desired benefit categories without maximizing the effect of one benefit and simultaneously negatively impacting one or more of the other benefits.

Accordingly it is an object of the invention to formulate a composition that remains on keratin surfaces for extended time periods, preferably more than one day, and more preferably over multiple days, where the drawbacks of flaking and poor wear and adhesion are minimized or eliminated and the product coated onto the keratin surface remains glossy.

### Summary of the Invention

The invention is directed to a multiple day wear composition as defined in claim 1 for keratin surfaces comprising at least one volatile solvent, at least one crosslinked organosiloxane film former, and from 5-12, preferably from 8.5 to 10.0% of montmorillonite minerals comprising a mixture of bentonite and hectorite wherein the ratio of bentonite to hectorite montmorillonite mineral ranges from 2 to 4, preferably from 2.5 to 3.5 parts of bentonite to 1 part of hectorite.

The invention is also directed to a method as defined in claim 12 for treating keratin surfaces to color or protect for a period of more than 1 day comprising applying to the treatment surface a composition comprising multiple day wear composition for keratin surfaces comprising at least one volatile solvent, at least one crosslinked organosiloxane film former, and from 8.5 to 10.0% of montmorillonite minerals comprising a mixture of bentonite and hectorite wherein the ratio of bentonite to hectorite montmorillonite mineral ranges from 2.5 to 3.5 parts of bentonite to 1 part of hectorite.

### Detailed Description

All percentages mentioned herein are percentages by weight unless otherwise indicated.

The term "keratin surfaces" means skin, hair, or nails.

The term "keratin fibers" means hair such as eyelashes, eyebrows, or hair; or nails such as fingernails or toenails.

The term "multiple day wear" means that the composition remains on the keratin surface to which it was applied for 1, 2, or 3 days, possible even longer. The wear is best indicated by the percentage of the film that is removed after each of the days when the individual wearing the film conducts daily activities in the normal manner (such as showering, washing hands, and the like).

The composition is in the anhydrous form.

The composition is substantially free of waxes. This means that it is free of solid or semi-solid waxes from animal, vegetable, or mineral sources.

The composition of the invention may be in the form of a mascara, a touch up application to gray hairs to color gray, a composition for application to skin to mask the appearance of blemishes, discoloration, scars, or skin imperfections; lip tints, tattoos, and the like. The 3 day wear property of the composition makes it ideal for applications where the applied composition is desired to remain on the keratin surface for more than 1 day when the individual undertakes their normal daily activities.

### Volatile Solvents

The composition contains at least one volatile solvent, which can be in the form of silicones, or paraffinic hydrocarbons.

Volatile solvents in the silicone or paraffinic hydrocarbon category generally have a viscosity ranging from 5x10⁻⁷ to 5x10⁻⁶ m²/s (0.5 to 5 centistokes (cst)) at 25° C, and include linear silicones, cyclic silicones, branched silicones, paraffinic hydrocarbons, or mixtures thereof. The volatile solvent may be present in amounts ranging from about 0.1 to 95%, preferably 15 to 90%, more preferably from about 45-90%.

Cyclic silicones are one type of volatile silicone that may be used in the composition. Such silicones have the general formula: 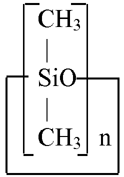 wherein=3-6, preferably 4, 5, or 6.

Also suitable are linear volatile silicones, for example, those having the general formula:

(CH₃)₃Si-O-[Si(CH₃)₂-O]ₙ-Si(CH₃)₃

wherein=0, 1, 2, 3, 4, or 5, preferably 0, 1, 2, 3, or 4.

Cyclic and linear volatile silicones are available from various commercial sources including Dow Corning Corporation and General Electric. The Dow Corning linear volatile silicones are sold under the trade names Dow Corning 244, 245, 344, and 200 fluids. These fluids include hexamethyldisiloxane (viscosity 0,65x10⁻⁶ m²/s (0,65 cst)), octamethyltrisiloxane (1,0x10⁻⁶ m²/s (1,0 cst)), decamethyltetrasiloxane (1,5x10⁻⁶ m²/s (1,5 cst)), dodecamethylpentasiloxane (2x10⁻⁶ m²/s (2,0 cst)) and mixtures thereof, with all viscosity measurements being at 25° C.

Suitable branched volatile silicones include alkyl trimethicones such as methyl trimethicone, a branched volatile silicone having the general formula: 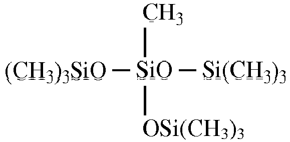 Methyl trimethicone may be purchased from Shin-Etsu Silicones under the tradename TMF-1.5, having a viscosity of 1,5x10⁻⁶m²/s (1,5 centistockes) at 25° C.

Also suitable as the volatile solvents are various straight or branched chain paraffinic hydrocarbons having 5, 6, 7, 8, 9, 10, 11, 12, 13, 14, 15, 16, 17, 18, 19, or 20 carbon atoms, more preferably 8 to 16 carbon atoms. Suitable hydrocarbons include pentane, hexane, heptane, decane, dodecane, tetradecane, tridecane, and C₈₋₂₀ isoparaffins as disclosed in U.S. Pat. Nos. 3,439,088 and 3,818,105, both of which are hereby incorporated by reference.

Preferred volatile paraffinic hydrocarbons have a molecular weight of 70-225, preferably 160 to 190 and a boiling point range of 30 to 320, preferably 60 to 260° C., and a viscosity of less than about 10x10⁻⁶ m²/s (10 cst) at 25° C. Such paraffinic hydrocarbons are available from EXXON under the ISOPARS trademark, and from the Permethyl Corporation. Suitable C₁₂ isoparaffins are manufactured by Permethyl Corporation under the tradename Permethyl 99A. Various C₁₆ isoparaffins commercially available, such as isohexadecane (having the tradename Permethyl R).

Most preferred is where the volatile solvent component comprises from 50-90%, preferably from about 55-85%, more preferably from about 60-80%. Further preferred is where this volatile solvent component comprises a mixture of volatile silicone and paraffinic hydrocarbon in a ratio of about 2 to 4 parts, preferably from 2.5 to 3.5, most preferably from 2.5 to 3.2 parts of volatile paraffinic hydrocarbon to volatile silicone.

### The Crosslinked Silicone Film Former

The composition comprises at least one crosslinked organosiloxane film former.

The crosslinked silicone film former used in the method and compositions of the invention may comprise the reaction product of a siloxane resin and a diorganosiloxane. Preferably, the amount of silicone copolymer in the compositions ranges from about 0.001 to 50%, preferably about 0.01-40%, more preferably 1-30% by weight of the total composition. This type of siloxane resin exhibits a plasticity that facilitates mixing and enables formulation with lesser amounts of oily plasticizers (which in turn reduce wear and adhesion).

Preferably, the siloxane resin is comprised of T or Q units, which may have M units and D units; and the diorganosiloxane is comprised of M and D units.

The term "M unit" means a monofunctional unit, which is a siloxy unit that contains one silicon atom bonded to one oxygen atom, with the remaining three substituents on the silicon atom being other than oxygen. In particular, in a monofunctional siloxy unit, the oxygen atom present is shared by 2 silicon atoms when the monofunctional unit is polymerized with one or more of the other units. In silicone nomenclature used by those skilled in the art, a monofunctional siloxy unit is designated by the letter "M", and means a unit having the general formula:

R₁R₂R₃-Si-O_{1/2}

wherein R₁, R₂, and R₃ are each independently C₁₋₃₀, preferably C₁₋₁₀, more preferably C₁₋₄ straight or branched chain alkyl, or C₁₋₃₀, preferably C₁₋₁₀, more preferably C₁₋₄ alkoxy, which may be substituted with phenyl or one or more hydroxyl groups; phenyl; carboxylic esters; or hydrogen. The SiO_{1/2} designation means that the oxygen atom in the monofunctional unit is bonded to, or shared, with another silicon atom when the monofunctional unit is polymerized with one or more of the other types of units. For example, when R₁, R₂, and R₃ are methyl the resulting monofunctional unit is of the formula I:

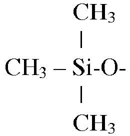

When this monofunctional unit is polymerized with one or more of the other units the oxygen atom will be shared by another silicon atom, i.e. the silicon atom in the monofunctional unit is bonded to 1/2 of this oxygen atom.

The term "difunctional siloxy unit" is generally designated by the letter "D" in standard silicone nomenclature. If the D unit is substituted with substituents other than methyl the "D" designation is sometimes used, which indicates a substituent other than methyl. For purposes of this disclosure, a "D" unit has the general formula:

R₁R₂-Si-O_{2/2}

wherein R₁ and R₂ are defined as above. The SiO_{2/2} designation means that the silicon atom in the difunctional unit is bonded to two oxygen atoms when the unit is polymerized with one or more of the other units. For example, when R₁ and R₂ are methyl the resulting difunctional unit is of the Formula II:

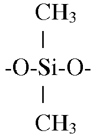

When this difunctional unit is polymerized with one or more of the other units the silicon atom will be bonded to two oxygen atoms, i.e. will share two one-halves of an oxygen atom.

The term "trifunctional siloxy unit" is generally designated by the letter "T" in standard silicone nomenclature. A "T" unit has the general formula:

R₁SiO_{3/2}

wherein R₁ is as defined above. The SiO_{3/2} designation means that the silicon atom is bonded to three oxygen atoms when the unit is copolymerized with one or more of the other units. For example when R₁ is methyl the resulting trifunctional unit is of Formula III:

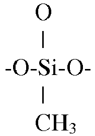

When this trifunctional unit is polymerized with one or more of the other units, the silicon atom shares three oxygen atoms with other silicon atoms, i.e. will share three halves of an oxygen atom.

The term "tetrafunctional siloxy unit" is generally designated by the letter "Q" in standard silicone nomenclature. A "Q" unit has the general formula:

Si-O_{4/2}

The SiO_{4/2} designation means that the silicon shares four oxygen atoms (i.e. four halves) with other silicon atoms when the tetrafunctional unit is polymerized with one or more of the other units. The SiO_{4/2} unit is best depicted as follows:

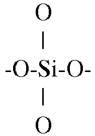

The silicone polymers used in the composition are made according to processes well known in the art. In general siloxane polymers are obtained by hydrolysis of silane monomers, preferably chlorosilanes. The chlorosilanes are hydrolyzed to silanols and then condensed to form siloxanes. For example, Q units are often made by hydrolyzing tetrachlorosilanes in aqueous or aqueous/alcoholic media to form the following: 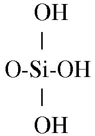 The above hydroxy substituted silane is then condensed or polymerized with other types of silanol substituted units including diorganosiloxane units, such as: 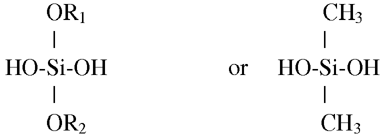 wherein R₁ and R₂ are as defined above.

Because the hydrolysis and condensation may take place in aqueous or aqueous/alcoholic media wherein the alcohols are preferably lower alkanols such as ethanol, propanol, or isopropanol, the units may have residual hydroxyl or alkoxy functionality. Preferably, the polymers are made by hydrolysis and condensation in aqueous/alcoholic media, which provides resins that have residual silanol and alkoxy functionality. In the case where the alcohol is ethanol, the result is a resin that has residual hydroxy or ethoxy functionality on the siloxane polymer. The silicone film forming polymers used in the compositions of the invention are generally made in accordance with the methods set forth in Silicon Compounds (Silicones), Bruce B. Hardman, Arnold Torkelson, General Electric Company, Kirk-Othmer Encyclopedia of Chemical Technology, Volume 20, Third Edition, pages 922-962, 1982, which is hereby incorporated by reference in its entirety.

If desired, the hydroxy functional groups on the molecule may be further reacted to form alkoxy groups, alkyl groups, halogens, which may be substituted with one or more substituents such as hydroxyl, and so on.

Most preferred is where the siloxane copolymer is obtained by reacting a diorganosiloxane having terminal hydroxyl groups with a siloxane resin having hydroxyl groups by combining the reactants in the presence of heat and ammonia, as set forth in U.S. Pat. No. 4,584,355, which is herby incorporated by reference in its entirety.

Particularly preferred are silicone copolymers manufactured by Dow Corning which are sold under the series 4100, 4200, 4300, 4400, 4500 or 4600. Most preferred are the Dow Corning silicone copolymers sold under the DC7-4405 trade name having the CTFA name dimethicone silylate which is referred to by the chemical name trimethylated silica treated with dimethicone.

### Montmorillonite Minerals (Clay)

Compositions according to the invention contain montmorillonite minerals, also referred to as clay. The invention compositions comprise from 5 to 12%, more preferably from 8.0 to 10.5%, more preferably from about 8.2 to 10.2%, most preferably from about 8.5 to 10.3% of clay. The compositions comprise a mixture of bentonite and hectorite wherein the ratio of bentonite to hectorite ranges from 2-4 parts of bentonite to 1 part of hectorite. More preferred is where the total clay is present in a ratio of from 2.5 to 3.5, preferably from 2.5 to 3.4, most preferably from about 2.57 to 3.32 parts of bentonite clay to 1 part of hectorite clay.

The bentonite is selected from those having CTFA names Hydrogenated tallowalkonium bentonite, Quaternium-18 bentonite, Benzalkonium bentonite, Quaternium-90 bentonite, Stearalkonium bentonite, or mixtures thereof. A suggested range for the bentonite clay is from 6 to 10% of the claimed composition.

The hectorite is selected from those having the CTFA names Dihydrogenated tallow benzylmonium hectorite, Disteardimonium hectorite, Quaternium-18 hectorite, Stearalkonium hectorite, or mixtures thereof. Suggested ranges for the hectorite clay are from about 1.5 to 3.5%, preferably from about 1.75 to 3.0%, most preferred from about 2.0 to 3.0%.

It is also possible for one or both of the clays to be present in the form of platelets or sheets that may or may not be interconnected. If desired the clay may be sheared to form nanoparticles or nanoplatelets, or sheets, of very small thickness. In such case the nanoplatelets have thicknesses ranging from about 0.5 to 5 nanometers (0.0005 to 0.005 microns). Preferably, the top surface area of the nanoplatelet ranges from about 20 to 2000 nanometers (0.02 to 2 microns).

In one preferred embodiment of the invention the clays are present in the form of dispersions with volatile or non-volatile, preferably volatile solvents. In one particularly preferred embodiment, the clays are quaternized montmorillonite minerals, more specifically Quaternium-90 bentonite which may be purchased in the form of a dispersion containing 2.5% propylene carbonate, 17.5 Quaternium-90 bentonite, and 80% isododecane sold under the tradename Distinctive Gel ID by D.C. Incorporated, Plainfield, NJ. The bentonite form may be present in the form of a dispersion containing propylene carbonate (3.5 parts)/disteardimonium hectorite (9.5 parts)/isododecane (87 parts) that is commercially available from Elementis Specialties under the tradename Bentone Gel EUGV.

### Particulate Materials

The composition of the invention preferably contains particulate materials in the form of pigments, inert particulates, or mixtures thereof. This is particularly desirable when the composition is intended to impart color. If present, suggested ranges are from about 0.01-75%, preferably about 0.5-70%, more preferably about 0.1-65% by weight of the total composition. In the case where the composition may comprise mixtures of pigments and powders, suitable ranges include about 0.01-75% pigment and 0.1-75% powder, such weights by weight of the total composition.

### A. Powders

The particulate matter may be colored or non-colored (for example white) non-pigmented powders. Suitable non-pigmented powders include bismuth oxychloride, titanated mica, fumed silica, spherical silica, polymethylmethacrylate, micronized teflon, boron nitride, acrylate copolymers, aluminum silicate, aluminum starch octenylsuccinate, calcium silicate, cellulose, chalk, corn starch, diatomaceous earth, fuller's earth, glyceryl starch, hydrated silica, kaolin, magnesium aluminum silicate, magnesium trisilicate, maltodextrin, microcrystalline cellulose, rice starch, silica, talc, mica, titanium dioxide, zinc laurate, zinc myristate, zinc rosinate, alumina, attapulgite, calcium carbonate, calcium silicate, dextran, kaolin, nylon, silica silylate, silk powder, sericite, soy flour, tin oxide, titanium hydroxide, trimagnesium phosphate, walnut shell powder, or mixtures thereof. The above mentioned powders may be surface treated with lecithin, amino acids, mineral oil, silicone, or various other agents either alone or in combination, which coat the powder surface and render the particles more lipophilic in nature.

### B. Pigments

The particulate materials may comprise various organic and/or inorganic pigments. The organic pigments are generally various aromatic types including azo, indigoid, triphenylmethane, anthroquinone, and xanthine dyes which are designated as D&C and FD&C blues, browns, greens, oranges, reds, yellows, etc. Organic pigments generally consist of insoluble metallic salts of certified color additives, referred to as the Lakes. Inorganic pigments include iron oxides, ultramarines, chromium, chromium hydroxide colors, and mixtures thereof. Iron oxides of red, blue, yellow, brown, black, and mixtures thereof are suitable.

In the most preferred embodiment the composition comprises 0.1 to 10%, preferably from about 0.5 to 8%, more preferably from about 1 to 6% of pigments.

The composition may contain a variety of other ingredients including but not limited to preservatives, humectants, anti-foam agents, and so on so long as these ingredients do not interfere with the optimal benefits for the composition. Such ingredients include preservatives, stabilizers for the montmorillonite minerals (e.g. propylene carbonate), additional film formers, and other ingredients.

The invention will be further described in connection with the following examples which are set forth for the purposes of illustration only.

### EXAMPLE 1

A composition according to the invention was made as follows:

| Ingredient | Wt% |
|---|---|
| Isododecane | QS100 |
| Trisiloxane | 18.00 |
| Dimethicone silylate | 12.00 |
| Quaternium-90 bentonite | 7.00 |
| Iron oxides | 4.90 |
| Disteardimonium hectorite | 2.40 |
| Propylene carbonate | 1.87 |
| Phenoxyethanol | 0.10 |
| Triethoxycaprylylsilane | 0.10 |
| | |
| Propylene carbonate (3.5 parts)/disteardimonium hectorite (9.5 parts)/isododecane (87 parts) | 25.00 |
| Propylene carbonate (2.5)/quaternium-90 bentonite (15.)/isododecane (80) | 39.90 |
| Trisiloxane/dimethicone silylate (60/40) | 30.00 |
| Phenoxyethanol | 0.10 |
| Iron oxides/triethoxycaprylylsilane (98/2) | 5.00 |

The composition was prepared by combining 25 parts of a hectorite gel composition (a mixture of 3.5 parts propylene carbonate, 9.5 parts disteardimonium hectorite and 87 parts isododecane); 39.90 parts of a bentone gel composition (a mixture of 2.5 parts propylene carbonate 15 parts quaternium-90 bentonite, and 80 parts isododecane), 30 parts of mixture of 40 parts of dimethicone silylate and 60 parts of trisiloxane; 0.1 part phenoxyethanol; and 5.0 parts iron oxides coated with triethoxycaprylylsilane and mixing well. The resulting composition had a viscosity of about 20-50 PaS at room temperature.

### EXAMPLE 2

Compositions according to the invention and comparative formulas were prepared with the following ingredients, and by combining all and mixing well to form a pourable liquid. Examples 1, 8-9 and 11-12 do not fall within the scope of the invention as claimed.

| Ingredient | 1 | 2 | 3 | 4 | 5 | 6 |
|---|---|---|---|---|---|---|
| Isododecane | 74.463 | 78.32 | 63.6500 | 53.6700 | 71.67 | 53.6700 |
| Trisiloxane | | | 9.0000 | 18.0000 | | 12.0000 |
| Dimethicone Silylate | 12.00 | 8.00 | 12.0000 | 12.0000 | 12.00 | 9.0000 |
| Disteardi-Monium Hectorite | 6.1655 | 1.9 | 2.3750 | 2.3750 | 2.375 | 2.3750 |
| Quaternium-90 bentonite | -- | 5.2325 | 6.1250 | 6.9825 | 6.9825 | 6.9825 |
| Iron oxides | 4.9000 | 4.9000 | 4.9000 | 4.9000 | 4.9000 | 4.9000 |
| Propylene carbonate | 2.2715 | 1.4475 | 1.7500 | 1.8725 | 1.8725 | 1.8725 |
| Phenoxy-Ethanol | 0.1000 | 0.1000 | 0.1000 | 0.1000 | 0.1000 | 0.1000 |
| Triethoxy-Caprylyl-Silane | 0.1000 | 0.1000 | 0.1000 | 0.1000 | 0.1000 | 0.1000 |
| | | | | | | |

| Ingredient | 7 | 8 | 9 | 10 | 11 | 12 |
|---|---|---|---|---|---|---|
| Isododecane | 53.6700 | 52.0650 | 71.6500 | 57.7500 | 81.7200 | 29.4000 |
| Trisiloxane | 15.0000 | 18.0000 | -- | 14.9400 | -- | 18.0000 |
| Dimethicone Silylate | 15.0000 | 12.0000 | 12.0000 | 9.9600 | -- | 12.0000 |
| Disteardi-Monium Hectorite | 2.3750 | 9.3750 | 9.3750 | 2.3750 | 3.8000 | 27.0000 |
| Quaternium-90 bentonite | 6.9825 | -- | -- | 7.8750 | 6.9825 | -- |
| Iron oxides | 4.9000 | 4.9000 | 4.9000 | 4.9000 | 4.9000 | 4.9000 |
| Propylene carbonate | 1.8725 | 3.4600 | 1.8750 | 2.000 | 2.3975 | 8.5000 |
| Phenoxy-Ethanol | 0.1000 | 0.1000 | 0.1000 | 0.1000 | 0.1000 | 0.1000 |
| Triethoxy-Caprylyl-Silane | 0.1000 | 0.1000 | 0.1000 | 0.1000 | 0.1000 | 0.1000 |

### EXAMPLE 3

Formulas 1-12 from Example 2 were tested for viscosity at 24 hours and 1 week; stability at 50° C. for 24 hours and 1 week; hardness, bending force in grams, adhesion in grams, gloss and contact angle.

Hardness was measured by drawing down a 2 mil [0.051 mm] film on a 2x3 inch [5.08 x 7.62 cm] glass plate. The film was allowed to dry overnight (24 hours). A Sward Hardness Rocker was placed on the dried film. The instrument was set to 50 oscillations in 60 +/-5 seconds. The hardness was calculated by multiplying the number of oscillations x 100/50 = % hardness.

Adhesion was measured by drawing down a 2 mil [0.051 mm] film on a 2x3 inch [5.08 x 7.62 cm] glass plate. The film was allowed to dry overnight (24 hours). The glass plate was then attached to the vertical wall of a TA.XT Plus Texture Analyzer wit Adhesion instrument attached. The Texture Analyzer was then arranged to scrape the film from the glass plate using a razor blade and the average force of the adherence of the film to the glass was measured in grams. The greater the numerical value, the greater the adhesion to the substrate.

Gloss was measured by drawing down a 2 mil [0.051 mm] film on a BYK opacity chart and allowing the film to dry overnight (24 hours). A micro-TRI-gloss instrument was placed on the film and turned on. The gloss data was read out on the digital display with surfaces measured at a 60° angle of incidence. Semi-gloss surfaces are read using an angle of incidence of 60°. and will generally provide readings in the range of 10-70 gloss units. Highly reflective surfaces that have gloss readings of greater than 70 gloss units when measured at 60° angle of incidence should be re-measured at 20°. Matte surfaces with measurements at 60° of less than 10 gloss units should be re-measured at 85°. The greater the numerical value, the glossier the film.

Bending force was measured using a TA.XT Plus Texture Analyzer with Bending Force instrument attached. A VitroSkin strip of 12x1 [30.5 x 2.54 cm] inches was coated with a 2 mil [0.051 mm] thickness film. The strip was inserted into the Analyzer so that it formed a loop with the largest portion of the loop toward the plate and the ends of the strip held together and affixed to the machine. The loop was lowered to the plate and the force required to bend the VitroSkin strip containing the film was noted. The force required to bend the VitriSkin strip with no film attached (baseline) value was subtracted from the measured value.

Viscosity was measured using a viscometer with readings taken at room temperature (25° C.).

Contact angle was measured by preparing a 2 mil [0.051 mm] draw down film on a 2x3 inch [5.08 x 7.62 cm] glass plate. The film was allowed to dry overnight to 24 hours. The slide was then placed on the OCA instrument table and a water droplet of 0.2 to 0.4 µl was dropped from the syringe onto the film substrate. The contact angle was measured and expressed in degrees.

The results for the tests performed are set forth below.

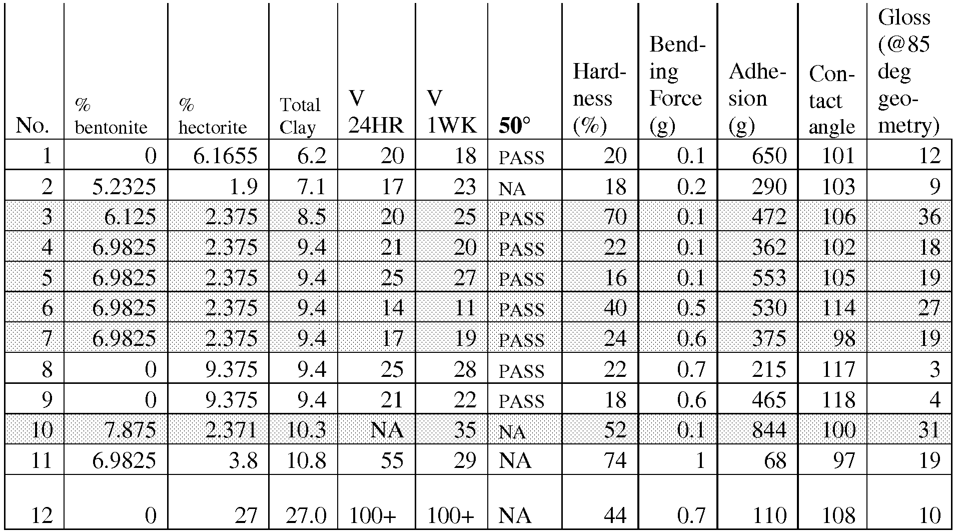

The most desirable formulas were those that exhibited good values for gloss, adhesion, bending force, and hardness, stability at 50° C. for up to one week. Since it was desired that the preferred composition be waterproof, the greater the contact angle the greater the hydrophobicity. In contrast, a formula that exhibited excellent results for one or two of these parameters but not all was not considered to be acceptable.

The results show that Formulas 3-7 and 10 provided optimized benefits in all measured categories. Specifically, when the total clay concentration of the formulas ranges between about 8.5 and 10.3 and the ratio of bentonite to hectorite is from about 2.5 to 3.5 all of the benefits are optimized and not at the expense of one or more of the individual benefits.

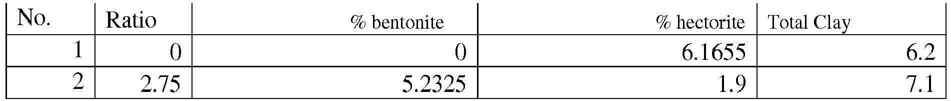

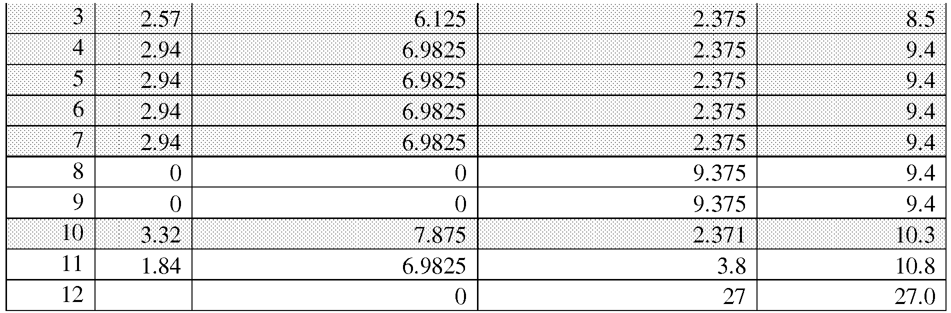

The above results show the criticality of the total clay concentration and the criticality of the ratio between the bentonite and hectorite.

### EXAMPLE 4

The formula of Example 1, according to the invention, was tested against a commercial brand advertised for having 3 days of wear. The ingredient list on this commercial product is reproduced below:
Water. Actylates Copolymer, Glyceryl Stearate, Disteardimomium Hectorite, Propylene Glycol, Stearic Acid, Cera Carnauba, Alcohol Denat., Triethanolamine, Synthetic Wax. Polyvinyl Alcohol, Lecithin, Propylene Carbonate, Tocopherol, Panthenol, Ascorbyl Palmitate, Glycine Soja Oil, Glycerin. Nylon-6, Xanthan Gum, Methylcellulose, Simethicone, Sodium Lareth-12 Sulfate, Oleic Acid, Glyceryl Oleate, Silica, Tetrasodium EDTA, Disodium EDTA, EDTA, Phosphoric Acid, Potassium Sorbate, Phenoxyethanol, Benzyl Alcohol, Ethylparaben, Methylparaben, Propylparaben, [+/-CI 77499, CI 77491, CI 77492, CI 77266]

The Example 1 formula and the commercial test product were applied to lashes of panelists. The amount of the composition remaining on the lashes was measured at 1, 2, and 3 days (24, 48, and 72 hours) by trained evaluators. The results are set forth below:

| Formula tested | % removed from lashes at 1 day | % removed from lashes at 2 days | % removed from lashes at 3 days |
|---|---|---|---|
| Example 1 | 7 | 14 | 22 |
| Commercial test formula | 23 | 28 | 31 |

The results demonstrate that the Example 1 Formula according to the invention provides optimal wear when compared to a commercially available product that is said to wear for three days.

## Claims

1. A multiple day wear composition for keratin surfaces comprising at least one volatile solvent, at least one crosslinked organosiloxane film former, and from 5 to 12% by weight of montmorillonite minerals comprising a mixture of bentonite and hectorite wherein the ratio of bentonite to hectorite montmorillonite mineral ranges from 2 to 4 parts of bentonite to 1 part of hectorite;
wherein the composition is anhydrous and free of animal, vegetable or mineral waxes; wherein the bentonite is selected from the group consisting of hydrogenated tallowalkonium bentonite, Quaternium-18 bentonite, Benzalkonium bentonite, Quaternium-90 bentonite, Stearalkonium bentonite, and mixtures thereof; and
wherein the hectorite is selected from the group consisting of Dihydrogenated tallow benzylmonium hectorite, Disteardimonium hectorite, Quaternium-18 hectorite, Stearalkonium hectorite, and mixtures thereof.

2. The composition of claim 1 wherein the volatile solvent comprises from 45-90% by weight of the total composition.

3. The composition of claim 2 wherein the volatile solvent comprises a mixture of volatile paraffinic hydrocarbon and volatile silicone in a ratio of 2 to 4 parts volatile paraffinic hydrocarbon to 1 part volatile silicone.

4. The composition of claim 3 wherein the volatile paraffinic hydrocarbon comprises isododecane and the volatile silicone comprises trisiloxane.

5. The composition of claim 1 wherein the crosslinked organosiloxane film former is obtained by reacting a diorganosiloxane having terminal hydroxyl groups with a siloxane resin having hydroxyl groups by combining the reactants in the presence of heat and ammonia.

6. The composition of claim 5 wherein the crosslinked organosiloxane film former is present from 1-30% by weight of the total composition.

7. The composition of claim 6 wherein the crosslinked organosiloxane film former is dimethicone silylate.

8. The composition of claim 1 wherein the bentonite comprises Quaternium-18 bentonite.

9. The composition of claim 1 wherein the hectorite is Disteardimonium hectorite.

10. The composition of claim 1 which wears for at least 2 to 3 days.

11. The composition of claim 1 comprising, by weight of the total composition:
45-90% of a volatile solvent comprising a mixture of paraffinic hydrocarbon and silicone in a ratio of 2 to 4 parts paraffinic hydrocarbon to 1 part silicone;
1-30% dimethicone silylate
8.5 to 10.0% of montmorillonite minerals comprising a mixture of bentonite and hectorite wherein the ratio of bentonite to hectorite montmorillonite mineral ranges from 2.5 to 3.5 parts of bentonite to 1 part of hectorite, preferably wherein the composition is in the form of a mascara, hair color touch up, or a touch up for masking the appearance of scars or skin imperfections.

12. A method for treating keratin surfaces to color or protect for a period of more than 1 day comprising applying to the treatment surface a composition comprising multiple day wear composition for keratin surfaces comprising at least one volatile solvent, at least one crosslinked organosiloxane film former, and from 8.5 to 10.0% by weight of montmorillonite minerals comprising a mixture of bentonite and hectorite wherein the ratio of bentonite to hectorite montmorillonite mineral ranges from 2.5 to 3.5 parts of bentonite to 1 part of hectorite, wherein the composition is anhydrous and free of animal, vegetable or mineral waxes;
wherein the bentonite is selected from the group consisting of hydrogenated tallowalkonium bentonite, Quaternium-18 bentonite, Benzalkonium bentonite, Quaternium-90 bentonite, Stearalkonium bentonite, and mixtures thereof; and
wherein the hectorite is selected from the group consisting of Dihydrogenated tallow benzylmonium hectorite, Disteardimonium hectorite, Quaternium-18 hectorite, Stearalkonium hectorite, and mixtures thereof.

13. The method of claim 12 wherein the composition remains on the keratin surface for 2 or 3 days, preferably wherein the composition remains on the keratin surface for 3 days.

14. The method of claim 12 wherein the composition is in the form of a mascara, a hair color touch up, or a product for masking the appearance of scars or skin imperfections.

15. The method of claim 12 wherein the composition comprises 50-90% by weight volatile solvent and 1-30% by weight of the crosslinked organosiloxane film former.

## Patentansprüche

1. Mehrere Tage haltende Zusammensetzung für Keratinoberflächen, umfassend mindestens ein flüchtiges Lösungsmittel, mindestens einen vernetzten Organosiloxanfilmbildner und von 5 bis 12 Gew.-% Montmorillonitmineralien, die eine Mischung aus Bentonit und Hectorit umfassen, wobei das Verhältnis von Bentonit- zu Hectorit-Montmorillonitmineral von 2 bis 4 Teilen Bentonit zu 1 Teil Hectorit reicht;
wobei die Zusammensetzung wasserfrei und frei von tierischen, pflanzlichen oder mineralischen Wachsen ist;
wobei das Bentonit ausgewählt ist aus der Gruppe, bestehend aus hydriertem Tallowalkoniumbentonit, Quaternium-18-Bentonit, Benzalkoniumbentonit, Quaternium-90-Bentonit, Stearalkoniumbentonit und Mischungen davon; und
wobei das Hectorit ausgewählt ist aus der Gruppe, bestehend aus dihydriertem Tallowbenzylmoniumhectorit, Disteardimoniumhectorit, Quaternium-18-Hectorit, Stearalkoniumhectorit und Mischungen davon.

2. Zusammensetzung nach Anspruch 1, wobei das flüchtige Lösungsmittel von 45-90 Gew.-% der Gesamtzusammensetzung umfasst.

3. Zusammensetzung nach Anspruch 2, wobei das flüchtige Lösungsmittel eine Mischung aus flüchtigem paraffinischem Kohlenwasserstoff und flüchtigem Silikon in einem Verhältnis von 2 bis 4 Teilen flüchtigem paraffinischem Kohlenwasserstoff zu 1 Teil flüchtigem Silikon umfasst.

4. Zusammensetzung nach Anspruch 3, wobei der flüchtige paraffinische Kohlenwasserstoff Isododecan umfasst und das flüchtige Silikon Trisiloxan umfasst.

5. Zusammensetzung nach Anspruch 1, wobei der vernetzte Organosiloxanfilmbildner durch Umsetzen eines Diorganosiloxans, das terminale Hydroxylgruppen aufweist, mit einem Siloxanharz, das Hydroxylgruppen aufweist, durch Kombinieren der Reaktanten in Gegenwart von Wärme und Ammoniak erhalten wird.

6. Zusammensetzung nach Anspruch 5, wobei der vernetzte Organosiloxanfilmbildner in einem Anteil von 1-30 Gew.-% der Gesamtzusammensetzung vorhanden ist.

7. Zusammensetzung nach Anspruch 6, wobei der vernetzte Organosiloxanfilmbildner Dimethiconsilylat ist.

8. Zusammensetzung nach Anspruch 1, wobei das Bentonit Quaternium-18-Bentonit umfasst.

9. Zusammensetzung nach Anspruch 1, wobei das Hectorit Disteardimoniumhectorit ist.

10. Zusammensetzung nach Anspruch 1, die mindestens 2 bis 3 Tage hält.

11. Zusammensetzung nach Anspruch 1, umfassend, bezogen auf das Gewicht der Gesamtzusammensetzung:
45-90 Gew.-% eines flüchtigen Lösungsmittels, umfassend eine Mischung aus paraffinischem Kohlenwasserstoff und Silikon in einem Verhältnis von 2 bis 4 Teilen paraffinischem Kohlenwasserstoff zu 1 Teil Silikon;
1-30 Gew.-% Dimethiconsilylat;
8,5 bis 10,0 Gew.-% Montmorillonitmineralien, die eine Mischung aus Bentonit und Hectorit umfassen, wobei das Verhältnis von Bentonit- zu Hectorit-Montmorillonitmineral von 2,5 bis 3,5 Teilen Bentonit zu 1 Teil Hectorit reicht, wobei die Zusammensetzung vorzugsweise in Form einer Mascara, einer Haarfarbenauffrischung oder einer Retusche zur Abdeckung des Auftretens von Narben oder Hautunregelmäßigkeiten vorliegt.

12. Verfahren zum Behandeln von Keratinoberflächen, um sie für einen Zeitraum von mehr als 1 Tag zu färben oder zu schützen, umfassend das Aufbringen einer Zusammensetzung auf die Behandlungsoberfläche, die eine mehrere Tage haltende Zusammensetzung für Keratinoberflächen umfasst, umfassend mindestens ein flüchtiges Lösungsmittel, mindestens einen vernetzten Organosiloxanfilmbildner und von 8,5 bis 10,0 Gew.-% Montmorillonitmineralien, die eine Mischung aus Bentonit und Hectorit umfassen, wobei das Verhältnis von Bentonit- zu Hectorit-Montmorillonitmineral von 2,5 bis 3,5 Teilen Bentonit zu 1 Teil Hectorit reicht, wobei die Zusammensetzung wasserfrei und frei von tierischen, pflanzlichen oder mineralischen Wachsen ist;
wobei das Bentonit ausgewählt ist aus der Gruppe, bestehend aus hydriertem Tallowalkoniumbentonit, Quaternium-18-Bentonit, Benzalkoniumbentonit, Quaternium-90-Bentonit, Stearalkoniumbentonit und Mischungen davon; und
wobei das Hectorit ausgewählt ist aus der Gruppe, bestehend aus dihydriertem Tallowbenzylmoniumhectorit, Disteardimoniumhectorit, Quaternium-18-Hectorit, Stearalkoniumhectorit und Mischungen davon.

13. Verfahren nach Anspruch 12, wobei die Zusammensetzung 2 oder 3 Tage auf der Keratinoberfläche verbleibt, wobei die Zusammensetzung vorzugsweise 3 Tage auf der Keratinoberfläche verbleibt.

14. Verfahren nach Anspruch 12, wobei die Zusammensetzung in Form einer Mascara, einer Haarfarbenauffrischung oder eines Produkts zur Abdeckung des Auftretens von Narben oder Hautunregelmäßigkeiten vorliegt.

15. Verfahren nach Anspruch 12, wobei die Zusammensetzung 50-90 Gew.-% flüchtiges Lösungsmittel und 1-30 Gew.-% des vernetzten Organosiloxanfilmbildners umfasst.

## Revendications

1. Composition pouvant être portée pendant plusieurs jours pour des surfaces kératiniques comprenant au moins un solvant volatil, au moins un agent filmogène à base d'organosiloxane réticulé, et de 5 à 12 % en poids de minéraux de type montmorillonite comprenant un mélange de bentonite et d'hectorite dans laquelle le rapport de minéraux de type montmorillonite bentonite à hectorite se situe dans la plage de 2 à 4 parties de bentonite pour 1 partie d'hectorite ;
dans laquelle la composition est anhydre et exempte de cires animale, végétale ou minérale ;
dans laquelle la bentonite est sélectionnée à partir du groupe constitué par la bentonite de tallowalkonium hydrogénée, la Quaternium-18 bentonite, la bentonite de benzalkonium, la Quaternium-90 bentonite, la bentonite de stéaralkonium, et des mélanges de celles-ci ; et
dans laquelle l'hectorite est sélectionnée à partir du groupe constitué par l'hectorite de benzylmonium de suif dihydrogénée, I hectorite de Distéardimonium, la Quaternium-18 hectorite, l'hectorite de Stéaralkonium, et des mélanges de celles-ci.

2. Composition selon la revendication 1, dans laquelle le solvant volatil comprend de 45 à 90 % en poids de la composition totale.

3. Composition selon la revendication 2, dans laquelle le solvant volatil comprend un mélange d'hydrocarbure paraffinique volatil et de silicone volatil dans un rapport de 2 à 4 parties d'hydrocarbure paraffinique volatil pour 1 partie de silicone volatil.

4. Composition selon la revendication 3, dans laquelle l'hydrocarbure paraffinique volatil comprend de l'isododécane et le silicone volatil comprend du trisiloxane.

5. Composition selon la revendication 1, dans laquelle l'agent filmogène à base d'organosiloxane réticulé est obtenu en faisant réagir un diorganosiloxane ayant des groupes hydroxyle terminaux avec une résine de siloxane ayant des groupes hydroxyle en combinant les réactifs en présence de chaleur et d'ammoniac.

6. Composition selon la revendication 5, dans laquelle l'agent filmogène à base d'organosiloxane réticulé est présent pour 1 à 30 % en poids de la composition totale.

7. Composition selon la revendication 6, dans laquelle l'agent filmogène à base d'organosiloxane réticulé est un silylate de diméthicone.

8. Composition selon la revendication 1, dans laquelle la bentonite comprend de la Quaternium-18 bentonite.

9. Composition selon la revendication 1, dans laquelle l'hectorite est la hectorite de Distéardimonium.

10. Composition selon la revendication 1, qui se porte pendant au moins 2 à 3 jours.

11. Composition selon la revendication 1 comprenant, en poids de la composition totale :
de 45 à 90 % d'un solvant volatil comprenant un mélange d'hydrocarbure paraffinique et de silicone dans un rapport de 2 à 4 parties d'hydrocarbure paraffinique pour 1 partie de silicone ;
de 1 à 30 % de silylate de diméthicone ;
de 8,5 à 10,0 % de minéraux de type montmorillonite comprenant un mélange de bentonite et d'hectorite dans lequel le rapport de minéraux de type montmorillonite bentonite à hectorite se situe dans la plage de 2,5 à 3,5 parties de bentonite pour 1 partie d'hectorite, de préférence dans laquelle la composition est sous la forme d'un mascara, d'une retouche pour couleur de cheveux, ou d'une retouche pour masquer l'apparition de cicatrices ou d'imperfections de la peau.

12. Procédé de traitement de surfaces kératiniques pour colorer ou protéger pendant une période de plus d'un jour, comprenant l'application, sur la surface de traitement, d'une composition comprenant une composition pouvant être portée pendant plusieurs jours pour des surfaces kératiniques comprenant au moins un solvant volatil, au moins un agent filmogène à base d'organosiloxane réticulé, et de 8,5 à 10,0 % en poids de minéraux de type montmorillonite comprenant un mélange de bentonite et d'hectorite dans laquelle le rapport de minéraux de type montmorillonite bentonite à hectorite se situe dans la plage de 2,5 à 3,5 parties de bentonite pour 1 partie d'hectorite, dans lequel la composition est anhydre et exempte de cires animale, végétale ou minérale ;
dans lequel la bentonite est sélectionnée à partir du groupe constitué par la bentonite de tallowalkonium hydrogénée, la Quaternium-18 bentonite, la bentonite de Benzalkonium, la Quaternium-90 bentonite, la bentonite de Stéaralkonium, et des mélanges de celles-ci ; et
dans lequel l'hectorite est sélectionnée à partir du groupe constitué par l'hectorite de benzylmonium de suif dihydrogénée, l'hectorite de distéardimonium, la Quaternium-18 hectorite, l'hectorite de Stéaralkonium, et des mélanges de celles-ci.

13. Procédé selon la revendication 12, dans lequel la composition reste sur la surface kératinique pendant 2 ou 3 jours, de préférence dans lequel la composition reste sur la surface kératinique pendant 3 jours.

14. Procédé selon la revendication 12, dans lequel la composition est sous la forme d'un mascara, d'une retouche pour couleur de cheveux, ou d'un produit pour masquer l'apparition de cicatrices ou d'imperfections de la peau.

15. Procédé selon la revendication 12, dans lequel la composition comprend de 50 à 90 % en poids de solvant volatil et de 1 à 30 % en poids de l'agent filmogène à base d'organosiloxane réticulé.
